# EUROPEAN PATENT APPLICATION

(11) **EP 2 151 198 A2**
(43) Date of publication of application: **10.02.2010**
(21) Application number: 09251933.9
(22) Date of filing: 04.08.2009
(51) Int. Cl.: A61B 17/06

(54) **Surgical needle with reduced contact area**

(30) Priority: 07.08.2008 US 187719
(71) Applicant: Tyco Healthcare Group LP, North Haven, CT 06473 (US)
(72) Inventor: Cabezas, Alan, Seymour CT 06483 (US)
(74) Representative: Soames, Candida Jane

(57) **Abstract**

The present disclosure relates to a surgical needle (200) that includes a proximal end adapted for attachment to a surgical filament (100), a distal end, and a shaft extending therebetween. An outer surface of the shaft includes reduction structure (210) configured and dimensioned to reduce contact between the outer surface and tissue without unnecessarily compromising the structural integrity or strength of the needle. Additionally, the configuration and dimensions of the reduction structure facilitate atraumatic insertion and passage of the surgical needle through the tissue during use.

## Description

### BACKGROUND

### 1. Technical Field

The present disclosure relates generally to apparatus for securing together tissue. More particularly, the present disclosure relates to a needle for use during surgical procedures including a reduced surface area to facilitate advancement of the needle through tissue.

### 2. Background of the Related Art

In surgical procedures, sutures are used to repair openings in skin, internal organs, blood vessels, and the like, as well as to join various tissues together. Generally, sutures are attached to a surgical needle that is forced through tissue to create an opening through which the suture may be drawn. However, contact between the surgical needle and the tissue can create forces of adhesion that can inhibit advancement of the needle, thus necessitating the application of increased force and potentially resulting in increased tissue trauma.

To address this concern, many surgical needles include structure that limits contact between the needle's outer surface and the tissue. For example, U.S. Patent No. 3,160,157 to Chisman (hereinafter "Chisman") and U.S. Patent No. 5,002,565 to McGregor (hereinafter "McGregor I") each describe surgical needles that include a plurality of raised edges or ribs defining recessed portions therebetween. U.S. Patent No. 5,853,423 to McGregor et al. (hereinafter "McGregor II") discusses a surgical needle that includes grooves defined in the outer surface. However, the edges discussed in both Chisman and McGregor I and the grooves described in McGregor II extend uninterruptedly along a substantial portion of the needle's length, thereby unnecessarily compromising the strength of the needle. Additionally, U.S. Patent Publication No. 2006/0173491 to Meade et al. (hereinafter "Meade") describes an arcuate suturing needle that includes a plurality of notches formed in an outer surface. However, the notches constitute abrupt, pronounced variations in the outer surface of the needle, and are configured and dimensioned to facilitate engagement with an external structure, such as a drive mechanism or a pin. Consequently, were the Meade needle to be used in an alternate application, e.g., without the drive mechanism, the notches may snag or catch the tissue, thereby resulting in unnecessary trauma.

Accordingly, a need remains in the art for a surgical needle including structure that facilitates atraumatic insertion of the needle through tissue by decreasing contact between the outer surface of the needle and the tissue, and thus, the amount of force required to advance the needle, without unnecessarily compromising the structural integrity or strength of the needle.

### SUMMARY

In one aspect of the present disclosure, a surgical needle is disclosed including a proximal end that is adapted for connection with a surgical filament, a distal end, and a shaft extending therebetween. An outer surface of the shaft includes reduction structure positioned intermittently along the length of the shaft. The reduction structure is configured and dimensioned to reduce contact between the surgical needle and tissue, and to facilitate atraumatic insertion and passage of the surgical needle through the tissue during use.

In one embodiment, the reduction structure includes at least one protrusion that extends outwardly from the outer surface of the shaft. The at least one protrusion may define any suitable geometric configuration, including but not limited to a substantially oval, substantially circular, substantially arcuate, or substantially linear configuration. In this embodiment of the reduction structure, the at least one protrusion may be configured such that the outer surface of the shaft is threaded. In an alternative embodiment, the at least one protrusion may include a plurality of protrusions that are spaced about a periphery of the shaft. In this embodiment, the plurality protrusions may be positioned uniformly, or randomly, about the outer surface of the shaft.

In an alternate embodiment of the reduction structure, the reduction structure includes at least one indentation extending inwardly from the outer surface of the shaft. The at least one indentation may define any suitable geometric configuration, including but not limited to a substantially oval, substantially circular, substantially arcuate, or substantially linear configuration. In one embodiment of the reduction structure, the at least one indentation may be configured such that the outer surface of the shaft is threaded. In an alternative embodiment, the at least one indentation may include a plurality of indentations that are spaced about a periphery of the shaft. In this embodiment, the plurality of protrusions may be positioned uniformly, or randomly, about the outer surface of the shaft.

In an alternate aspect of the present disclosure, a surgical needle assembly is disclosed that includes a surgical filament connected to a needle. The needle includes a shaft with an outer surface having at least one protrusion associated therewith. The at least one protrusion extends outwardly from the shaft and is configured and dimensioned to reduce contact between the shaft, and to facilitate atraumatic insertion and passage of the surgical needle through the tissue during use.

The at least one protrusion may include a plurality of protrusions spaced along a length of the shaft, and/or a plurality of protrusions spaced about a periphery of the shaft.

In another aspect of the present, a method of manufacturing a surgical needle is disclosed that includes the steps of providing an elongate member and altering an outer surface of the elongate member to include reduction structure positioned along the elongate member. The reduction structure is positioned intermittently along a length of the elongate member and is configured and dimensioned to reduce contact between the elongate member and tissue, and to facilitate atraumatic insertion and passage of the elongate member through the tissue during use.

### BRIEF DESCRIPTION OF THE DRAWINGS

Various embodiments of the present disclosure are described herein below with reference to the drawings, wherein:

FIG. 1 is a side, perspective view of a surgical needle assembly including a filament and a needle incorporating reduction structure in accordance one embodiment of the present disclosure;

FIG. 1A is a side, perspective view of an alternate embodiment of the needle seen in FIG. 1 exhibiting a substantially linear configuration;

FIG. 2 is a front, end view of the needle seen in FIG. 1;

FIG. 3 is a side, plan view of the needle seen in FIG. 1 including an alternate embodiment of the reduction structure;

FIG. 4 is a side, plan view of the needle seen in FIG. 1 including another embodiment of the reduction structure;

FIG. 5 is a front, end view of the needle seen in FIG. 4;

FIGS. 6-11 are side, plan views of the needle seen in FIG. 1 illustrating additional embodiments of the reduction structure;

FIG. 12 is a front, end view of the needle seen in FIG. 11;

FIGS. 13-19 are side, plan views of the needle seen in FIG. 1 illustrating additional embodiments of the reduction structure; and

FIG. 20 is a side, perspective view of the needle seen in FIG. 1 including an additional embodiment of the reduction structure.

### DETAILED DESCRIPTION

In the following description and in the accompanying drawings, in which like references numbers identify similar or identical elements, the term "proximal" should be understood as referring to the end of the presently disclosed surgical needle assembly, or any component thereof that is closest to a practitioner during proper use, while the term "distal" should be understood as referring to the end that is furthest from the practitioner during proper use. Additionally, the term "filament" should be understood as referring to any elongate member suitable for the intended purpose of joining tissue, including but not limited to sutures, ligatures, and surgical tape, and the term "tissue" should be understood as referring to any bodily tissue, including but not limited to skin, fascia, ligaments, tendons, muscle, and bone.

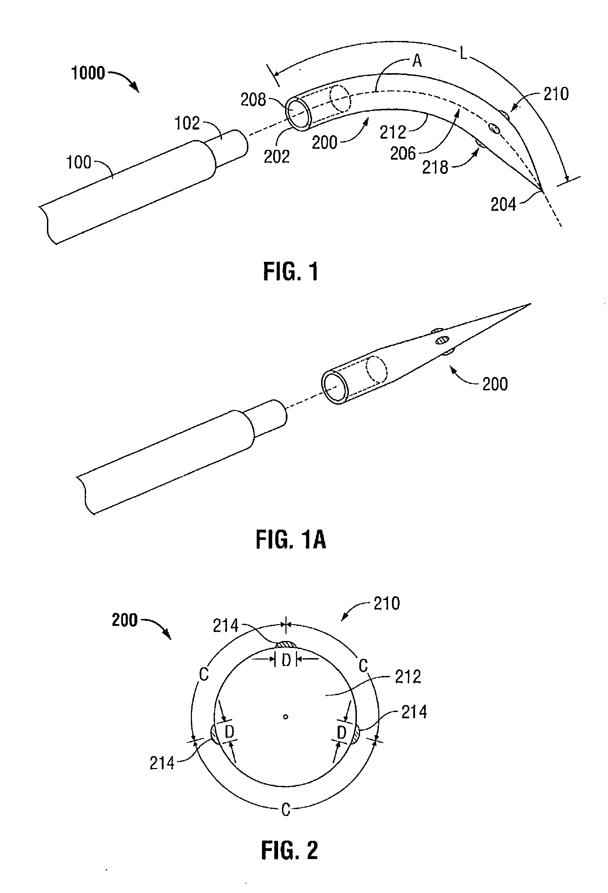

FIG. 1 illustrates one embodiment of a surgical needle assembly, referred to generally by reference character 1000, that includes a filament 100 and a needle 200 that is attachable to an end 102 of the filament 100. The filament 100 can be formed from any suitable biocompatible material of either the absorbable or non-absorbable variety, including but not limited to catgut, silk, nylon, polyesters, polyethylene, polypropylene, steel, polymeric and copolymeric materials, and stainless steel. Desirably, the filament 100 has a measure of flexibility such that the filament 100 can be manipulated by a practitioner to join adjacent sections of tissue together. As an illustrative example, the filament 100 may be used to repair or close an incision, wound, or the like using conventional suturing techniques.

With reference now to FIG. 2 as well, the needle 200 will be discussed. The needle 200 may be formed of any suitable biocompatible material, including but not limited to stainless steel or polymeric materials, and may define a configuration that is partially or wholly arcuate, as shown, or substantially linear, as seen in FIG. 1A.

The needle 200 is an elongate member that includes proximal and distal ends 202, 204, respectively, and a shaft 206 that extends therebetween. The proximal end 202 of the needle 200 is attachable, either releasably or fixedly to the end 102 of the filament 100, and may be attached thereto in any suitable manner. For instance, in the embodiment of the needle assembly 1000 illustrated in FIG. 1, the proximal end 202 of the needle 200 includes a bore 208 that is configured and dimensioned to receive the end 102 of the filament 100. In an alternate embodiment, however, the filament 100 and the needle 200 may be integrally formed.

The distal end 204 of the needle 200 may exhibit any configuration suitable for the intended purpose of facilitating the penetration of tissue. While the needle 200 is illustrated as including a distal end 204 that is pointed or incisive, alternatively, the distal end 204 may be substantially blunt.

The shaft 206 extends from the proximal end 202 to the distal end 204 along an axis "A" to define a length "L". The shaft 206 includes reduction structure 210 that extends away, i.e., outwardly of, or inwardly from, an outer surface 212 of the shaft 206. The reduction structure 210 is configured and dimensioned to create gradual, smooth topographical variations in outer surface 212 of the shaft 206 to decrease the surface area of the needle 200 that is in contact with tissue during use. By decreasing the surface area of the needle 200 that is in contact with tissue, the reduction structure 210 reduces the force necessary to advance the needle 200, and facilitates the atraumatic insertion and removal thereof.

In the embodiment of the reduction structure 210 seen in FIGS. 1 and 2, the reduction structure 210 includes one or more protrusions 214 extending outwardly of the outer surface 212 of the shaft 206. As illustrated, the reduction structure 210 includes three protrusions 214 that are spaced about the periphery of the needle 200. The protrusions 214 may be spaced equally from each other to define a distance "C" therebetween, as seen in FIGS. 1-2, or alternatively, the spacing between adjacent protrusions 214 may vary. The protrusions 214 may each define an equivalent dimension "D" as shown, or alternatively, may vary in size. In additional embodiments of the reduction structure 210, the protrusions 214 may be present in fewer or greater numbers and/or may be arranged differently, as described below.

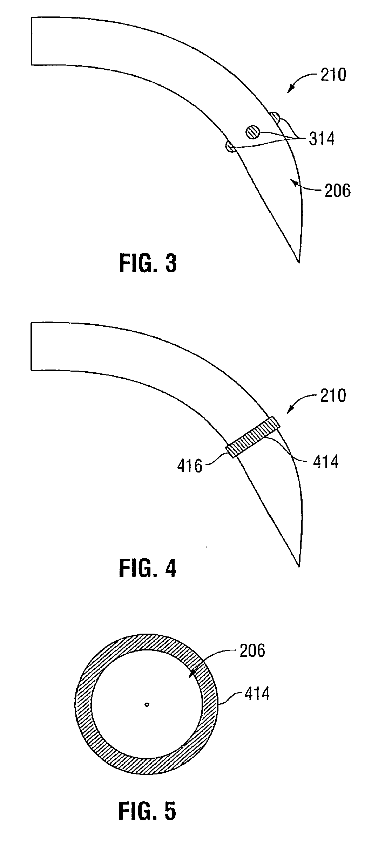

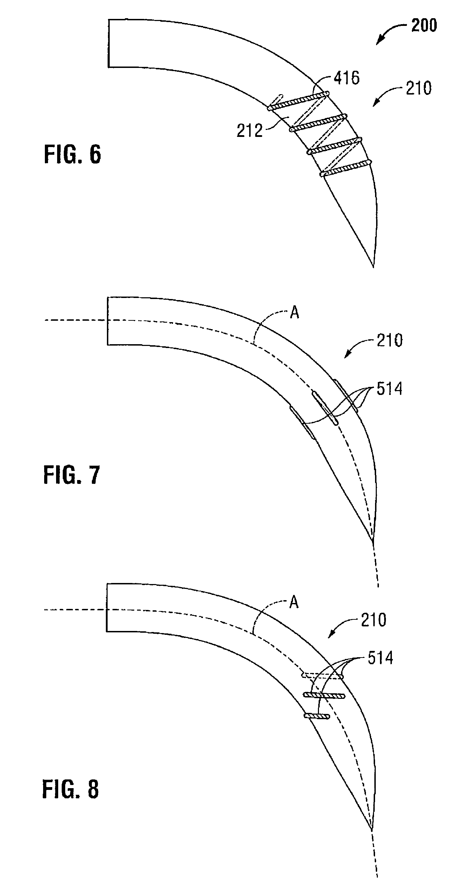

Although illustrated as substantially oval in configuration, the protrusions 214 may exhibit any other suitable geometric configuration. For example, the reduction structure 210 may include protrusions 314 that are substantially circular, as seen in FIG. 3. Instead, the reduction structure 210 may include one or more protrusions 414 having an arcuate configuration such that the protrusions 210 extend continuously about the periphery of the shaft 206 to define one or more ribs 416, as seen in FIGS. 4-5. The reduction structure 210 may alternatively include one or more protrusions 416 attributing a threaded configuration to the outer surface 212 of the needle 200, as seen in FIG. 6. In another variation, the reduction structure 210 may include protrusions 514 that are substantially linear in configuration, as seen in FIG. 7. The linear protrusions 514 may extend along the axis "A", as seen in FIG. 7, or alternatively, may extend in transversely in relation thereto, as seen in FIG. 8. While the reduction structure 210 has been described and illustrated as including only a single variety of protrusion in the embodiments seen in FIGS. 1-8, it should be appreciated that alternate embodiments of the reduction structure 210 may include any suitable combination of the aforedescribed protrusions.

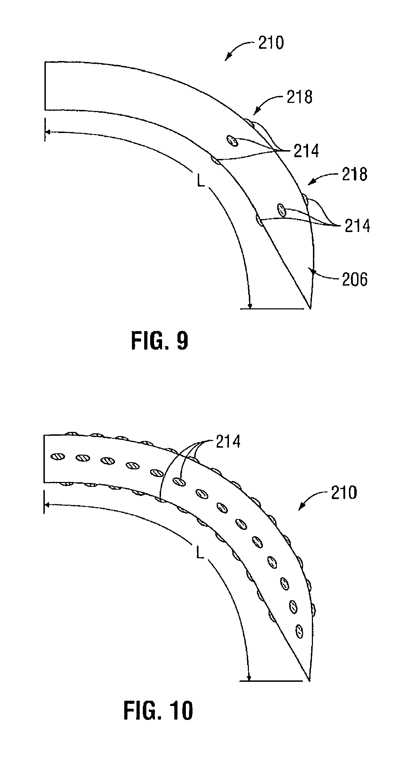

Referring again to FIG. 1, the protrusions 214 may be are arranged about the periphery of the shaft 206 to define a single band 218. Alternatively, however, the protrusions 210 may be arranged to define a plurality of bands 218 that are spaced along the length "L" of the shaft 206, as seen in FIG. 9, or the protrusions 214 may be positioned uniformly along the length "L" of the shaft, as seen in FIG. 10.

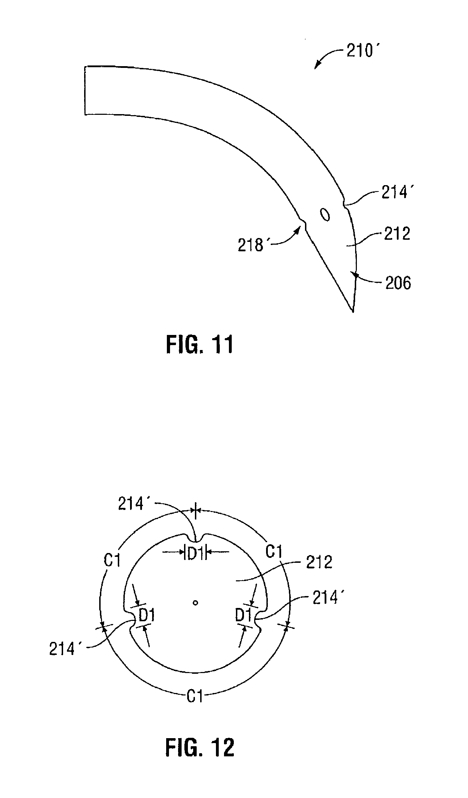

Referring now to FIGS. 11-19, another embodiment of the reduction structure, referred to generally by reference character 210', will be discussed. In this embodiment, the reduction structure 210' includes one or more indentations 214' extending inwardly from the outer surface 212 of the shaft 206.

In the embodiment of the reduction structure 210' seen in FIGS. 11-12, the reduction structure 210' is illustrate as including three indentations 214' that are spaced about the periphery of the needle 200. The indentations 214' may be spaced equally from each other to define a distance "C1" therebetween, as seen in FIGS. 11-12, or alternatively, the spacing between adjacent indentations 214' may vary. The indentations 214' may each define an equivalent dimension "D1" as shown, or alternatively, may vary in size. In additional embodiments of the reduction structure 210', the indentations 214' may be present in fewer or greater numbers and/or may be arranged differently, as described below.

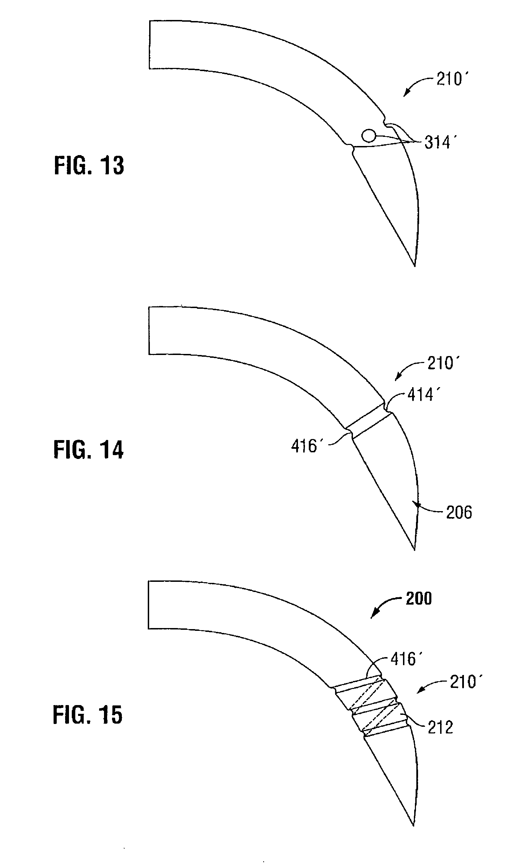

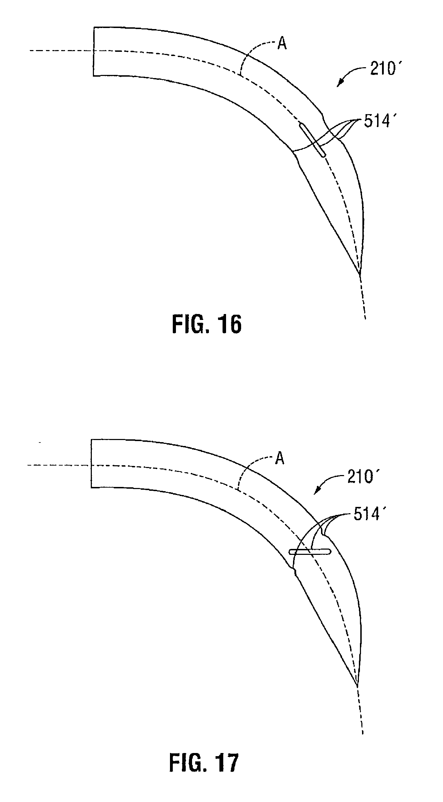

While the indentations 214' are illustrated as substantially oval on configuration, any other suitable geometric configuration may be employed. For example, the reduction structure 210' may include indentations 314' that are substantially circular, as seen in FIG. 13. Instead, the reduction structure 210' may include indentations 414' having an arcuate configuration such that the indentations 414' extend continuously about the periphery of the shaft 206 to define one or more channels 416', as seen in FIG. 14. The reduction structure 210' may alternatively include one or more indentations 416' attributing a threaded configuration to the outer surface 212 of the needle 200, as seen in FIG. 15. In another variation, the reduction structure 210 may include indentations 514' that are substantially linear in configuration, as seen in FIG. 16. The linear indentations 514' may extend along the axis "A", as seen in FIG. 16, or alternatively, may extend transversely in relation thereto, as seen in FIG. 17.

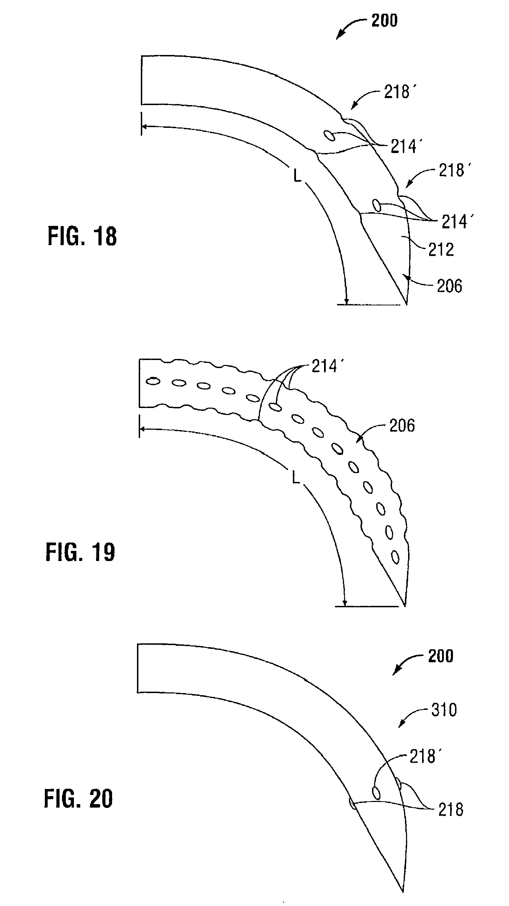

Referring again to FIG. 11, the reduction structure 210' may include indentations 214' that are arranged about the periphery of the shaft 206 to define a single band 218'. Alternatively, however, the indentations 214' may be arranged to define a plurality of bands 218' that are spaced along the length "L" of the shaft 206, as seen in FIG. 18, or the indentations 214' may be positioned uniformly along the length "L" of the shaft 206, as seen in FIG. 19.

FIG. 20 illustrates the needle 200 including another embodiment of the reduction structure, referred to generally by reference character 310. In this embodiment, the reduction structure 310' includes a combination of protrusions and indentations. Although illustrated as including the protrusions 218 discussed above with respect to FIG. 1 and the indentations 218' discussed above with respect to FIG. 11, in alternate embodiments of the reduction structure 310, any of the aforedescribed protrusions or indentations may be included, either alone or in combination.

The reduction structure 210, 210', 310 described above with reference to FIGS. 1-10, FIGS. 11-19, and FIG. 20, respectively, may be formed through any suitable method of manufacture. Referring to FIG. 18 as an example, the indentations 214' of the reduction structure 210' may be formed by grinding or milling the outer surface 212 of the needle 202, e.g., through the use of mechanical apparatus or lasers, or alternatively, the needle 200 may be cast in a die configured and dimensioned to form the indentations 214'.

Persons skilled in the art will understand that the devices and methods specifically described herein and illustrated in the accompanying drawings are intended to be construed as non-limiting, exemplary embodiments, and that the features illustrated or described in connection with one exemplary embodiment may be combined with the features of other embodiments. Additionally, one skilled in the art will appreciate further features and advantages of the disclosure based on the above-described embodiments. As such, the disclosure is not to be limited by what has been particularly shown and described, except as indicated by the appended claims.

## Claims

1. A surgical needle comprising:
a proximal end adapted for attachment to a surgical filament, a distal end, and a shaft extending therebetween, wherein an outer surface of the shaft includes reduction structure positioned intermittently along a length of the shaft, the reduction structure being configured and dimensioned to reduce contact between the surgical needle and tissue, wherein the reduction structure is configured and dimensioned to facilitate atraumatic insertion and passage of the surgical needle through the tissue during use.

2. The surgical needle of claim 1, wherein the reduction structure includes at least one protrusion extending outwardly from the outer surface of the shaft.

3. The surgical needle of claim 2, wherein the at least one protrusion includes a plurality of protrusions spaced about a periphery of the shaft; or the surgical needle of claim 2, wherein the at least one protrusion defines a substantially oval configuration; or the surgical needle of claim 2, wherein the at least one protrusion defines a substantially circular configuration; or the surgical needle of claim 2, wherein the at least one protrusion defines a substantially arcuate configuration; or the surgical needle of claim 2, wherein the at least one protrusion defines a substantially linear configuration; or the surgical needle of claim 2, wherein the at least one protrusion is configured such that the outer surface of the needle defines at least a partial threaded configuration; or the surgical needle of claim 2, wherein the at least one protrusion includes a plurality of protrusions positioned uniformly about the outer surface of the shaft.

4. The surgical needle of claim 1, wherein the reduction structure includes at least one indentation extending inwardly from the outer surface of the shaft.

5. The surgical needle of claim 4, wherein the at least one indentation includes a plurality of indentations spaced about a periphery of the shaft; or the surgical needle of claim 4, wherein the at least one indentation defines a substantially oval configuration; or the surgical needle of claim 4, wherein the at least one indentation defines a substantially circular configuration; or the surgical needle of claim 4, wherein the at least one indentation defines a substantially arcuate configuration; or the surgical needle of claim 4, wherein the at least one indentation defines a substantially linear configuration; or the surgical needle of claim 4, wherein the at least one indentation is configured such that the outer surface of the needle defines a threaded configuration; or the surgical needle of claim 4, wherein the at least one indentation includes a plurality of indentations positioned uniformly about the outer surface of the shaft.

6. A surgical needle assembly, comprising:
a surgical filament; and
a needle attached to the surgical filament and including a shaft with an outer surface having at least one protrusion associated therewith, the at least one protrusion extending outwardly from the shaft and being configured and dimensioned to reduce contact between the shaft and tissue, wherein the at least one protrusion is configured and dimensioned to facilitate atraumatic insertion and passage of the surgical needle through the tissue during use.

7. The surgical needle of claim 6, wherein the at least one protrusion includes a plurality of protrusions spaced along a length of the shaft.

8. The surgical needle of claim 6, wherein the at least one protrusion includes a plurality of protrusions spaced about a periphery of the shaft.

9. A method of manufacturing a surgical needle, comprising the steps of:
providing an elongate member; and
altering an outer surface of the elongate member to include reduction structure positioned along a length of the elongate member, the reduction structure being positioned intermittently along a length of the elongate member and configured and dimensioned to reduce contact between the elongate member and tissue, wherein the reduction structure is configured and dimensioned to facilitate atraumatic insertion and passage of the surgical needle through the tissue during use.
